(19) Europäisches Patentamt
European Patent Office
Office européen des brevets

(11) Veröffentlichungsnummer: **0 147 811**
**B1**

# (12) EUROPÄISCHE PATENTSCHRIFT

(45) Veröffentlichungstag der Patentschrift: **16.08.89**

(51) Int. Cl.⁴: **A 61 K 31/535**, A 61 K 47/00

(21) Anmeldenummer: **84116004.7**

(22) Anmeldetag: **20.12.84**

## (54) Photostabilisierung von Molsidomin.

(30) Priorität: **23.12.83 DE 3346638**

(43) Veröffentlichungstag der Anmeldung: **10.07.85 Patentblatt 85/28**

(45) Bekanntmachung des Hinweises auf die Patenterteilung: **16.08.89 Patentblatt 89/33**

(84) Benannte Vertragsstaaten: **AT BE CH DE FR GB IT LI NL**

(56) Entgegenhaltungen:
**EP-A- 0 074 620**
**EP-A- 0 127 468**
**DE-A- 3 230 273**

**CHEMICAL ABSTRACTS, Band 101, Nr. 4, Juli 1984, Seite 296, Zusammenfassung Nr. 28312f, Columbus, Ohio, US; & JP-A-59 55 822**
**CHEMICAL ABSTRACTS, Band 94, 1981, Seite 511, Zusammenfassung Nr. 190531k, Columbus, Ohio, US; F. IKAWA: "Studies on natural food coloring agens. 13. of riboflavin"**

(73) Patentinhaber: **CASSELLA Aktiengesellschaft, Hanauer Landstrasse 526, D-6000 Frankfurt am Main 61 (DE)**

(72) Erfinder: **Voegele, Dieter, Kurt-Schumacher-Ring 16, D-6454 Bruchköbel (DE)**
Erfinder: **Laudenbach, Petra, Rodaustrasse 33, D-6052 Mühlheim (DE)**

(74) Vertreter: **Urbach, Hans-Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 147 811 B1

ACTORUM AG

**Beschreibung**

Die vorliegende Erfindung betrifft die Stabilisierung von Molsidomin («Index Nominum 1980», Centre scientifique de la Société suisse de pharmacie, Zürich (1979), Seite 569) gegen Licht sowie Molsidomin enthaltende Mischungen, die durch hohe Lichtstabilität ausgezeichnet sind und die als Lichtstabilisator eine Verbindung aus der Reihe der Flavonole oder der Flavanone bzw. der korrespondierenden Hydroxychalcone («Index Nominum 1980», Centre scientifique de la Société suisse de pharmacie, Zürich (1979), Seite 881) enthalten.

Es ist eine seit langem bekannte und in vielen Bereichen der Technik häufig beobachtete Erscheinung, daß chemische Verbindungen durch Lichteinwirkung in ihrer Struktur verändert oder zerstört werden. Besonders bekannt ist das Ausbleichen von Farbstoffen und das Verspröden von Kunststoffen durch Licht. Besonders unangenehm und auch gefährlich sind die durch Licht ausgelösten chemischen Veränderungen in pharmakologisch wirksamen Substanzen. Hier kann die Lichteinwirkung zu einer Abschwächung der erwünschten Wirkung und/oder zum Auftreten neuer unter Umständen schädlicher Wirkungen führen. Wegen der damit verbundenen erheblichen Gefahren für den mit solchen pharmazeutischen Substanzen behandelten Patienten ist es von fundamentaler Bedeutung, diese pharmakologisch wirksamen, als Medikament eingesetzten Verbindungen gegen durch Licht ausgelöste chemische Veränderungen zu schützen.

Lichtschutz ist in vielen Bereichen der Technik erforderlich und wird auf verschiedenen Wegen praktiziert. So ist es bekannt, lichtempfindliche Stoffe in lichtundurchlässige Verpackungen einzuschließen. Es ist auch bekannt, solche Umhüllungen einzusetzen, die teildurchlässig sind und die den Teil der Lichtstrahlung absorbieren, der die schädigende Wirkung entfaltet. Hierbei wird von der einleuchtenden Annahme Gebrauch gemacht, daß nur solche Strahlungsanteile zu einer chemischen Veränderung der Verbindung führen können, die von dieser Verbindung auch absorbiert werden. In diesem Sinne ist auch bereits vorgeschlagen worden, die zu schützenden Substanzen direkt mit dem Lichtschutzmittel zu überziehen. In die Gruppe dieser Ausführungsformen des Lichtschutzes fallen z.B. Lichtschutzanstriche aber auch kosmetische Zubereitungen mit Lichtschutzmitteln. Es ist auch bekannt, lichtempfindlichen Kunststoffen Lichtschutzmittel beizumengen und schließlich ist, aufbauend auf Untersuchungen über die Photolyse des Nifedipins (Rainhard Klimek, Inaugural-Dissertation, Frankfurt(Main), 1978) auch vorgeschlagen worden, pharmakologisch wirksame Substanzen dadurch vor Lichtschädigung zu schützen, daß man sie mit Lösungen von lichtabsorbierenden Stoffen mischt und entweder in Form der lichtgeschützten Lösungen verwendet oder aus den Lösungen durch Entzug des Lösungsmittels feste, lichtstabilisierte Zubereitungen herstellt. Hierbei sollen als lichtabsorbierende Stoffe solche eingesetzt werden, die, insbesondere in dem stabilitätsgefährdeten Bereich von 300 bis 440 nm, ein ähnliches Absorptionsverhalten aufweisen wie das zu schützende Pharmakon.

Dieser bekannte Vorschlag ist jedoch nicht verallgemeinerungsfähig. So ist es beispielsweise gezeigt worden, daß Nifedipin («Index Nominum 1980», Centre scientifique de la Société suisse de pharmacie, Zürich (1979), Seite 596) durch 8-Hydroxychinolin und durch gelbe Lebensmittelfarbstoffe, d.h. Verbindungen, die im Bereich der spectralen Absorption des Nifedipins ebenfalls starke Absorptionsbande aufweisen, sehr wirksam gegen Photolyse geschützt werden kann. Untersucht man dagegen den lichtinduzierten Abbau des Nifedipins in Gegenwart von Troxerutin, so zeigt sich kein nennenswerter Schutzeffekt, obgleich die Absorptionskurven dieser beiden Substanzen sich ebenfalls sehr ähneln und insbesondere in dem als besonders kritisch angesehenen Bereich von 300 bis 440 nm Absorptionsmaxima aufweisen, die nur ca. 10 nm auseinander liegen.

Hieraus schien sich zu ergeben, daß Troxerutin trotz theoretisch brauchbaren Absorptionsverhaltens aus anderen, bisher unbekannten Gründen zur Photostabilisierung von lichtempfindlichen Stoffen unbrauchbar ist. Es war daher nicht vorherzusehen, daß Troxerutin sich hervorragend zur Photostabilisierung von Molsidomin eignet. Dieser Befund ist um so überraschender als im Hinblick auf die bisherigen Vorstellungen über Lichtschutzwirkung Troxerutin das Molsidomin auch deshalb gar nicht schützen sollte, weil die Absorptionsmaxima dieses Substanzpaares im Bereich von 300 bis 440 nm viel weiter, nämlich ca. 40 nm, auseinander liegen als bei Nifedipin und Troxerutin.

Es wurde nun weiter gefunden, daß die Photostabilisierung von Molsidomin nicht auf die Verwendung von Troxerutin beschränkt ist, sondern daß sich zu diesem Zweck Verbindungen der Formel I

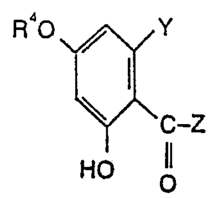

(I)

worin

Y eine Hydroxygruppe, Alkoxy mit 1 oder 2 C-Atomen oder β-Hydroxyethoxy und

Z eine Gruppe der Formel

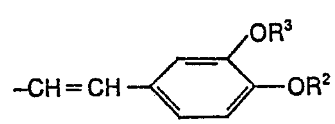

oder

Y und Z gemeinsam ein Strukturelement der Formel II oder III bedeuten

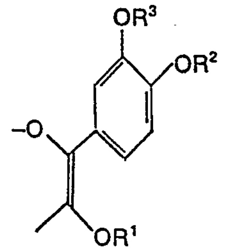

(II)

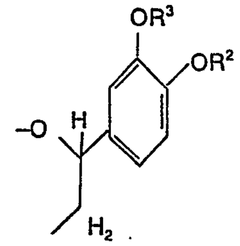

(III)

dessen freie Sauerstoffvalenz in der Y-Position und dessen freie Kohlenstoffvalenz in der Z-Position gebunden ist, und

$R^1$ Wasserstoff oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist, und Ester dieser Verbindungen mit pharmakologisch unbedenklichen Säuren sowie gegebenenfalls deren Salze mit pharmakologisch unbedenklichen Basen hervorragend eignen.

Die vorliegende Erfindung betrifft auch photostabilisierte Mischungen von Molsidomin, einem oder mehreren Photostabilisatoren der Formel I und gegebenenfalls weiteren Mischungskomponenten, wie z.B. Lösungsmitteln, Dispergiermitteln, Hilfs- und Zusatzstoffen. Insbesondere betrifft die vorliegende Erfindung auch photostabilisierte pharmazeutische Zubereitungen von Molsidomin, die neben dem Wirkstoff als Photostabilisator eine oder mehrere Verbindungen der Formel I sowie weitere in der Galenik übliche Hilfs- und Zusatzstoffe enthalten.

Die Verbindungen der Formel I zeigen alle, ähnlich dem Troxerutin, eine überraschende, hohe Lichtschutzwirkung für Molsidomin, die im Hinblick auf die relativ schwache Lichtabsorption dieser Verbindungen in keiner Weise zu erwarten waren.

Bevorzugt für den erfindungsgemäßen Einsatz sind solche Verbindungen der Formel I, in denen Y, Z, $R^1$, $R^2$ und G die obengenannten Bedeutungen haben und $R^3$ Wasserstoff oder β-Hydroxyethyl und $R^4$ Wasserstoff, β-Hydroxyethyl oder ein Rest G eines Mono- oder Disaccharids ist. Bevorzugt sind auch solche Verbindungen der Formel I, in denen eines der Symbole $R^1$ oder $R^4$ einen Rest G bedeutet.

Besonders bevorzugt sind solche Verbindungen der Formel I, die mehrere bevorzugte Merkmale aufweisen. Besonders bevorzugt sind beispielsweise Hesperidin-methyl-chalcon, Hesperidin-phosphat, Hesperidin, Quercetin, Hesperetin, Quercitrin, Rutin, Rutin-sulfat und insbesondere Troxerutin.

Soweit die Verbindungen der Formel I freie OH-Gruppen aufweisen, können diese mit pharmakologisch unbedenklichen Säuren verestert werden. Zur Veresterung geeignete Säuren sind z.B. niedere aliphatische Carbonsäuren, wie Ameisensäure, Essigsäure, Propionsäure, Buttersäure, Milchsäure, Maleinsäure, Bernsteinsäure, Äpfelsäure, Weinsäure oder anorganische Säure, wie Schwefelsäure und insbesondere Phosphorsäure. Bei der Veresterung mit mehrbasischen Säuren werden die 1:1-Veresterungsprodukte hergestellt, bei denen jeweils nur eine der Säurefunktionen verestert, die übrigen frei sind. Bevorzugte Ester sind solche, die in der Molsidomin-Mischung eine bessere Löslichkeit haben als die unveresterte Verbindung.

Sowohl die schwach sauren aromatischen Hydroxylgruppen der erfindungsgemäßen Verbindungen der Formel I als auch die freien Säurefunktionen der sauren Ester der Verbindungen der Formel I mit mehrbasischen Säuren können durch Einwirkung von pharmakologisch unbedenklichen Basen in die Salze überführt werden. Auch diese Salze zeigen die hohe Lichtschutzwirkung der Verbindungen der Formel I und sind daher zum erfindungsgemäßen Einsatz gut geeignet. Sie können gegenüber den unveresterten Verbindungen der Formel I auch noch anwendungstechnische Vorteile aufweisen. Wenn z.B. die freie Verbindung der Formel I aufgrund geringer Löslichkeit schlecht in eine wäßrige Zubereitung eingearbeitet werden kann, so kann der Einsatz eines Salzes mit erhöhter Wasserlöslichkeit, wie z.B. eines Alkalisalzes eines Phosphorsäuremonoesters, die Herstellung der Zubereitung erheblich erleichtern.

Die Verbindungen der Formel I können einzeln oder auch im Gemisch untereinander für den erfindungsgemäßen Zweck eingesetzt werden. Sofern in bestimmten galenischen Zubereitungen eine zu geringe Löslichkeit der erfindungsgemäß einzusetzenden Photostabilisatoren vorliegt, kann auch der Zusatz von Lösungsvermittlern und solubilisierend wirkenden Hilfsstoffen zweckmäßig sein. Solche, die gleichmäßige Verteilung von schwerlöslichen Komponenten in pharmazeutischen Zubereitungen begünstigende bzw. bewirkende Mittel sind aus der Literatur (z.B. H. Sucker, P. Fuchs, P. Speiser, Pharmazeutische Technologie, (1978) G. Thieme Verlag, Stuttgart, Seite 281 ff.) in großer Zahl bekannt. Beispiele für solche Hilfsstoffe sind polyoxethylierte Fettalkoholether, polyoxethylierte Sorbitan-Fettsäureester oder polyoxethylierte Stearinsäurepolyolester.

Die erfindungsgemäß einzusetzenden Verbindungen der Formel I sind entweder bekannt und zum Teil handelsüblich, oder sie lassen sich vom Fachmann durch einfache, bekannte Alkylierungs- oder Hydroxyethylierungsreaktionen oder durch hydrolytische Abspaltung eines Saccharidrestes aus bekannten Verbindungen herstellen. Es sind

auch verschiedene gute Methoden bekannt zur Herstellung dieser Verbindungen, ausgehend von Resorcin und Resorcinderivaten (vgl. hierzu die Lehrbücher der organischen Chemie z. B. Gabor Fodor, Organische Chemie, Bd. 2, VEB Deutscher Verlag der Wissenschaften, Berlin (1965); L. F. Fieser und M. Fieser, Lehrbuch der Organischen Chemie GmbH, Weinheim, Bergstraße (1957)).

So können die für $R^2$, $R^3$ und $R^4$ stehenden Niederalkylgruppen durch Umsetzung der korrespondierenden phenolischen Verbindungen in schwach alkalischem, vorzugsweise wäßrigem Medium mit bekannten Methylierungs- oder Ethylierungsmitteln, wie z. B. Dimethyl- oder Diethylsulfat, erhalten werden. Die entsprechenden Hydroxyethyl-Derivate können analog durch Umsetzung der phenolischen Verbindungen mit Ethylenoxid hergestellt werden. Eventuell anfallende Reaktionsgemische können durch bekannte Methoden, z. B. fraktioniertes Kristallisieren, vorzugsweise durch Chromatographie, getrennt werden; sie können aber ebensogut als Mischung für den erfindungsgemäßen Zweck eingesetzt werden. Sofern eine Synthese, ausgehend von Resorcin oder seinen Derivaten, gewünscht wird, kann durch Umsetzung der Ausgangsverbindung zum Trihydroxy- oder Trialkoxy-acetophenon und dessen Kondensation mit Dialkoxybenzaldehyd das gegebenenfalls entsprechend alkylierte Chalcon der Formel I dargestellt werden. Das freie Chalcon kann gewünschtenfalls durch Protonenkatalyse zum entsprechend substituierten Flavanonderivat der Formel I cyclisiert werden, und dieses wiederum gestattet, z. B. durch Umsetzung mit salpetriger Säure und anschließende Hydrolyse den Zugang in die Reihe der Flavone der Formel I.

Überraschenderweise ist die Lichtschutzwirkung von Verbindungen der Formel I für Molsidomin erheblich besser als die des als Lichtschutzmittel ebenfalls bereits bekannten Lebensmittelfarbstoffs Chinolingelb («Richtlinie für färbende Stoffe» vom 23. 10. 1962, E 104; Chinophthalonsulfonsäure). So ergibt sich z. B. für eine Lösung von 2 mg/ml Molsidomin in 0,9%iger NaCl-Lösung ohne Lichtschutzzusatz und Standard-Belichtung ein Regressionskoeffizient von −0,165, in Gegenwart von 10 mg/ml Troxerutin ein Regressionskoeffizient von −0,021 und in Gegenwart von 10 mg/ml Chinolingelb ein Regressionskoeffizient von −0,089.

Auch die Figur 1 veranschaulicht die nicht vorhersehbare, hervorragende Lichtschutzwirkung von Troxerutin auf Molsidomin. Sie zeigt den Abbau von Molsidomin in einer 0,9%igen NaCl-Lösung bei einer Anfangskonzentration von ca. 2 mg/ml Molsidomin ohne (Kurve 0) und in Gegenwart von 20 mg/ml (Kurve 1) Troxerutin unter natürlichen Belichtungsbedingungen (farbloses Glas, Südfenster, sonnig). Der Regressionskoeffizient, der sich aus diesen Abbaukurven ergibt, beträgt ohne Troxerutin −6,34 und mit Troxerutin −0,95 und gibt auch zahlenmäßig einen überzeugenden Beweis der ausgezeichneten Schutzwirkung.

Figur 2 zeigt die hohe Schutzwirkung von Troxerutin in einer Tablettenzubereitung unter standardisierten Belichtungsbedingungen (Suntest). Man sieht, daß ohne (Kurve 0) Troxerutin der Wirkstoff Molsidomin in der Tablette (8 mg) im Verlauf von 6 h auf 52,5% der ursprünglichen Menge abgebaut wird, während bereits bei einem Zusatz von nur 8 mg (Kurve 1) Troxerutin der Abbau so verlangsamt wird, daß nach 6 h noch 91,3% des Wirkstoffs erhalten sind und bei Einsatz von 80 mg (Kurve 2) Troxerutin praktisch kein merklicher Wirkstoffabbau mehr erfolgt.

Wird die Konzentration der Verbindungen der Formel I in Molsidomin-Lösungen schrittweise herabgesetzt und jeweils unter gleichen Belichtungsbedingungen der Molsidominabbau gemessen, so findet man überraschenderweise, daß die Lichtschutzwirkung mit der Konzentration des Troxerutins nur sehr langsam zurückgeht.

Ein Beispiel hierfür ist der belichtungsbedingte Abbau von Molsidomin in Retardtabletten mit 8 mg Wirkstoffgehalt ohne und in Gegenwart von 8 mg, 0,8 mg und 0,08 mg Troxerutin pro Tablette. Die Figur 3 zeigt die erhaltenen Meßwerte. Man erkennt, daß unter der Lichteinwirkung nach einer Anfangsphase, in der eine sprunghafte Abnahme der Molsidominmenge in der Tablette erfolgt, ein sehr gut linearer Abbau des Wirkstoffs eintritt. Extrapoliert man den linearen Kurventeil, so ergibt sich, daß die Molsidominmenge in der troxerutinfreien Tablette bereits nach 4,4 Std. auf 50% der Anfangsmenge gefallen ist, während bei der Tablette, die Troxerutin in einer Menge von nur $^1/_{100}$ der Wirkstoffmenge enthält, erst nach 10,2 Std. 50% des Wirkstoffs abgebaut sind. Hieraus ergibt sich, daß schon durch einen Zusatz von Molsidomin in einer Menge von $^1/_{100}$ der Wirkstoffmenge sich die Stabilität des Molsidomins in den Tabletten um den Faktor 2,3 erhöht.

Noch drastischer ist die Verbesserung der Lichtstabilität von Molsidomin durch Troxerutinzusatz in flüssigen Zubereitungen zu erkennen. Mißt man z. B. die Wirkstoffabnahme von Molsidomin-Tropfen, die 1,8 mg Molsidomin und Troxerutin in wechselnden Mengen zwischen 18 mg und 0,0018 (!) mg pro ml enthalten und die in Braunglasflaschen abgefüllt sind, in einem Suntest-Gerät, so ergeben sich die aus Fig. 4 ersichtlichen Konzentrationskurven. Hier zeigt auch noch eine Schutzstoffmenge von nur $^1/_{1000}$ der Wirkstoffmenge eine drastische Verbesserung der Lichtstabilität des Wirkstoffs: Mit dem genannten geringen Schutzstoffzusatz ist erst nach ca. 21,6 h die Wirkstoffkonzentration auf 50% der Ausgangskonzentration abgesunken, während ohne Zusatz von Schutzstoff dieser Abbauwert bereits nach 9,2 h erreicht ist. Dies entspricht einer Stabilitätsverbesserung um den Faktor 2,3. Bedenkt man, daß das Gewichtsverhältnis Molsidomin : Troxerutin von 1000:1 einem Molverhältnis von 3000:1 entspricht, so ist die mit einer so geringen Menge von Lichtschutzsubstanz erreichbare Stabilitätsverbesserung um den Faktor 2,3 völlig überraschend.

Zur Photostabilisierung werden die Verbindungen der Formel I dem Molsidomin im Mengenver-

hältnis 0,001:1 bis 50:1, vorzugsweise 0,1:1 bis 20:1 zugesetzt. In der Praxis, um eine Schutzreserve auch für ungünstige Belichtungsverhältnisse zu schaffen, kann es zweckmäßig sein, als Untergrenze dem Molsidomin die 0,3- bis 0,5fache Menge an Photostabilisator der Formel I zuzusetzen.

Eine Herabsetzung der Stabilisatormenge unter $^1/_{1000}$ der Molsidominmenge hat eine kontinuierliche Verminderung der Schutzwirkung zur Folge; sie hat dort ihre Grenze, wo die Schutzwirkung nicht mehr den Anforderungen gerecht wird. Noch niedrigere Stabilisatorzusätze sind daher als eine verschlechterte Ausführungsform der vorliegenden Erfindung zu betrachten. Das gleiche gilt für Zusätze, die deutlich über der oben angegebenen Höchstgrenze liegen. Solche hohen Zusätze haben keinen zusätzlichen praktischen Nutzen für die Stabilisierung gegenüber Licht.

In Molsidomin-Mischungen oder pharmazeutischen Molsidomin-Zubereitungen, die sehr niedrige Konzentrationen von Molsidomin enthalten, wird man zweckmäßigerweise nicht nur $^1/_{1000}$ der Molsidomin-Menge an Photostabilisatoren der Formel I zusetzen, da dies zu technisch schwer zu realisierenden Stabilisatorkonzentrationen führen würde und ein unzureichender Lichtschutz zu befürchten ist. Vorteilhafterweise werden daher in der Praxis den Molsidomin-Mischungen und -Zubereitungen Photostabilisatoren in einer Menge zugesetzt, die mindestens der 0,1fachen, vorzugsweise mindestens der 0,3fachen und insbesondere mindestens der 0,5fachen Menge des Molsidomins entsprechen. In der Regel ergeben sich hierbei in der Praxis Stabilisatorkonzentrationen von mindestens 0,062 bis 0,5 Gew%, vorzugsweise 0,19 bis 1,5 Gew%, insbesondere 0,31 bis 2,5 Gew%, bezogen auf das Gewicht der Gesamtmischung.

Die Photostabilisierung von reinem Molsidomin durch Zusatz einer oder mehrerer Verbindungen der Formel I, d.h. die Herstellung von Mischungen, die außer Molsidomin und dem Stabilisator keine weiteren Zusätze enthalten, kommt insbesondere dann in Betracht, wenn es gilt, Molsidomin bei der Herstellung, beim Versand, bei der Lagerung und bei der Weiterverarbeitung gegen Licht zu schützen.

Selbstverständlich kann das Molsidomin auch für nichtpharmazeutische Zwecke durch Zusatz von Verbindungen der Formel I stabilisiert werden. Je nach dem technischen Einsatzgebiet können dann neben Molsidomin und der Verbindung der Formel I auch noch andere Hilfs- oder Zusatzstoffe, wie Träger-, Netz- oder Dispergiermittel, evtl. auch Nahrungsstoffe, in den Mischungen enthalten sein.

Auch für den pharmazeutischen Einsatz werden in der Regel nicht die photostabilen Mischungen verwendet, die ausschließlich Molsidomin und eine oder mehrere Verbindungen der Formel I enthalten, sondern es werden entweder Lösungsmittel oder aber feste Füll-, Verdünnungs- oder Hilfsmittel, wie sie bei der Herstellung pharmazeutischer Zubereitungen in der Galenik üblich sind, in den Mischungen enthalten sein.

Die erfindungsgemäßen photostabilisierten Molsidomin-Zubereitungen können Wirkstoff und Photostabilisator der Formel I in fester und/oder gelöster Form enthalten.

Erfindungsgemäß durch Verbindungen der Formel I lichtstabilisierte pharmazeutische Zubereitungen können flüssig bis fest sein. Flüssige pharmazeutische Zubereitungen sind z.B. Lösungen wie sie für Injection oder Infusion oder für orale Applikation in Form von Tropfen eingesetzt werden. Solche erfindungsgemäßen Lösungen enthalten Molsidomin in Konzentrationen, die in der Regel je nach Applikationsart zwischen 0,01 mg/ml und 100 mg/ml liegen. Neben dem Molsidomin enthalten die Lösungen die erforderliche Konzentration an Verbindungen der Formel I, die, wie oben angegeben, das 0,001- bis 50fache der Molsidominkonzentration beträgt, sowie das Lösungsmittel, wie z.B. isotonische Kochsalzlösung, Wasser, andere übliche verträgliche Lösungsmittel bzw. Lösungsmittelgemische und gegebenenfalls weitere in der Galenik übliche Hilfsstoffe, oder, sofern es sich um ein Kombinationspräparat handelt, auch weitere pharmazeutische Wirkstoffe. Verdickte Lösungen, wie z.B. Pasten, Salben oder Pflaster, die zur transdermalen Therapie geeignet sind, können auch noch höhere Molsidominkonzentrationen aufweisen.

Feste Zubereitungen enthalten Molsidomin in Konzentrationen, die in der Regel je nach Zubereitungsform zwischen 0,1 mg/g und 50 mg/g liegen, die erforderliche Menge an Verbindungen der Formel I, die wie oben angegeben, das 0,001-, vorzugsweise 0,3- bis 50fache, insbesondere das 0,5- bis 20fache der Molsidominmenge beträgt, sowie die in galenischen Zubereitungen üblichen weiteren Zusätze, z.B. von Füllmitteln, Quellmitteln, Sprengmitteln, Gleitmitteln, Haftmitteln, Form- und Trennmitteln, Überzugsmitteln oder Kapselmaterialien usw., wie sie aus der Literatur bekannt sind, oder, sofern es sich um ein Kombinationspräparat handelt, auch weitere pharmazeutische Wirkstoffe.

Bei festen geformten Zubereitungen kann der erfindungsgemäße Lichtschutz auch dadurch erfolgen, daß man eine feste geformte Molsidomin-Zubereitung, die frei ist von Lichtschutzmitteln, mit einem Überzug versieht, der eine ausreichende Konzentration einer Verbindung der Formel I enthält.

Dieses Verfahren kommt vor allem für Tabletten, Pillen und Dragees in Betracht. Die erforderliche Konzentration des Photostabilisators in dem Überzug hängt vorwiegend von der normalerweise zu erwartenden Lichtbelastung des Präparates ab. In der Regel wird die Konzentration der Verbindungen der Formel I in dem Überzug zwischen 2 und 30 Gew%, vorzugsweise 10 bis 25 Gew%, bezogen auf die Überzugsmasse, gewählt.

Die für das konkrete Anwendungsgebiet geeignete Molsidominkonzentration kann in an sich bekannter Weise aus den bekannten Wirkungsdaten

des Molsidomins leicht berechnet werden. Sie liegen z.B.

für Infusionslösungen bei 0,002 mg/ml bis 0,4 mg/ml,

für Injektionslösungen bei 0,2 mg/ml bis 20 mg/ml,

für Tropfen bei 0,4 mg/ml bis 20 mg/ml,

für Tabletten, überzogene Tabletten und Kapseln bei 5 mg/g bis 200 mg/g.

Die Herstellung fester bis flüssiger pharmazeutischer Zubereitungen ist an sich bekannt durch eine umfassende Literatur. Die Herstellung der erfindungsgemäßen, lichtstabilisierten Molsidomin-Zubereitungen kann nach diesen, an sich bekannten Methoden erfolgen. Hervorzuheben ist lediglich, daß auch bei der Herstellung fester Zubereitungen alle bekannten Verfahren eingesetzt werden können. So ist es möglich aber nicht erforderlich, die Zubereitung durch Eindampfen einer Lösung der Bestandteile oder einer Mischung einiger fester Bestandteile und einer Lösung einer oder mehrerer Verbindungen der Formel I herzustellen. Die erfindungsgemäße Lichtschutzwirkung ergibt sich aber überraschenderweise auch, wenn alle Bestandteile in fester Form zusammengebracht und in bekannter Weise zu einer pharmazeutischen Zubereitung verarbeitet werden.

Der Einsatz von Verbindungen der Formel I als Lichtschutzmittel für Molsidomin ist von besonderem Vorteil, weil die Schutzwirkung sehr hoch ist, man also mit geringen Zusatzmengen einen ausreichenden Lichtschutz erhält. Gegenüber dem grundsätzlich auch möglichen Lichtschutz durch Lebensmittelfarbstoffe bietet der Einsatz von Verbindungen der Formel I neben der hohen Wirksamkeit jedoch noch einen zusätzlichen Vorteil:

Lebensmittelfarbstoffe müssen, wenn sie einen wirksamen Lichtschutz bieten sollen, in erheblich höherer Konzentration eingesetzt werden als sie üblicherweise für das Anfärben von Lebensmitteln verwendet werden. Da Lebensmittelfarbstoffe aber auch im Tagesverlauf mit den verschiedensten Nahrungsmitteln in nicht kontrollierten Mengen aufgenommen werden, könnte bei zusätzlicher Zufuhr in Arzneimitteln evtl. eine Annäherung oder gar eine Überschreitung der empfohlenen Tages-Höchstdosis dieser Farbstoffe erfolgen. Diese Gefahr wird bei Einsatz von Troxerutin als Lichtschutzstoff vermieden.

Zur Veranschaulichung der Erfindung seien die folgenden Ausführungsbeispiele angegeben.

Beispiel 1

Erfindungsgemäße, lichtstabilisierte Molsidomintropfen haben beispielsweise folgende Zusammensetzung:

| | |
|---|---|
| Molsidomin | 2 mg |
| Troxerutin | 15 mg |
| Methylparaben | 0,7 mg |
| Propylparaben | 0,3 mg |
| entmineralisiertes Wasser ad | 1 ml |

Herstellung

In 3 Liter entmineralisiertem Wasser werden bei Siedetemperatur 3,5 g Methylparaben und 1,5 g Propylparaben gelöst. Nach dem Abkühlen auf Zimmertemperatur werden in dieser Lösung 10 g Molsidomin und 75 g Troxerutin unter Rühren aufgelöst und das Volumen der Lösung durch Zusatz von entmineralisiertem Wasser auf 5 Liter eingestellt.

Beispiel 2

Eine erfindungsgemäße, lichtstabilisierte Molsidominlösung für Ampullen bzw. Infusionsampullen hat beispielsweise folgende Zusammensetzung:

| | |
|---|---|
| Molsidomin | 2 mg |
| Troxerutin | 2 mg |
| Wasser für Injektionszwecke | ad 1 ml |

Herstellung

In 3 Liter Wasser für Injektionszwecke werden 10 g Molsidomin und 10 g Troxerutin unter Rühren aufgelöst und das Volumen der Lösung durch Zusatz von Wasser für Injektionszwecke auf 5 Liter eingestellt.

Beispiel 3

Eine erfindungsgemäße, lichtstabilisierte Molsidominlösung für Ampullen bzw. Infusionsampullen hat beispielsweise folgende Zusammensetzung:

| | |
|---|---|
| Molsidomin | 2 mg |
| Troxerutin | 100 mg |
| Wasser für Injektionszwecke | ad 1 ml |

Herstellung

Die Herstellung erfolgt wie im Beispiel 2 beschrieben, mit dem Unterschied, daß anstelle der dort angegebenen Menge 500 g Troxerutin eingesetzt werden.

Beispiel 4

Eine erfindungsgemäße, lichtstabilisierte Molsidominlösung für Ampullen bzw. Infusionsampullen hat beispielsweise folgende Zusammensetzung:

| | |
|---|---|
| Molsidomin | 2 mg |
| Troxerutin | 40 mg |
| Wasser für Injektionszwecke | ad 1 ml |

Herstellung

Die Herstellung erfolgt wie im Beispiel 2 beschrieben, mit dem Unterschied, daß anstelle der dort angegebenen Menge 200 g Troxerutin eingesetzt werden.

Beispiel 5

Eine erfindungsgemäße, lichtstabilisierte Molsidomintablette hat beispielsweise folgende Zusammensetzung:

| | |
|---|---|
| Molsidomin | 2 mg |
| Troxerutin | 2 mg |
| Maisstärke | 40 mg |
| Milchzucker | 115 mg |
| Magnesiumstearat | 1 mg |

Herstellung

20 g Molsidomin, 20 g Troxerutin, 400 g Maisstärke, 1150 g Milchzucker und 10 g Magnesiumstearat werden in einem Mischaggregat homogen gemischt, und dann wird die Mischung zu Tabletten von 160 mg Gewicht verpreßt.

Beispiel 6

Eine erfindungsgemäße, lichtstabilisierte Molsidomintablette hat beispielsweise folgende Zusammensetzung:

| | |
|---|---|
| Molsidomin | 4 mg |
| Troxerutin | 40 mg |
| Maisstärke | 80 mg |
| Milchzucker | 194 mg |
| Magnesiumstearat | 2 mg |

Herstellung

40 g Molsidomin, 400 g Troxerutin, 1940 g Milchzucker und 600 g Maisstärke werden in einem Mischaggregat homogen gemischt. Diese Mischung wird mit einem Stärkekleister aus 60 g Maisstärke und 340 ml Wasser granuliert, über ein 1,1 mm-Sieb passiert und im Umlufttrockenschrank bei 60 °C getrocknet. Dem Granulat werden 140 g Maisstärke und 20 g Magnesiumstearat zugemischt, und die erhaltene Mischung wird zu Tabletten von 320 mg Gewicht verpreßt.

Beispiel 7

Eine erfindungsgemäße, lichtstabilisierte Molsidomintablette hat beispielsweise folgende Zusammensetzung:

| | |
|---|---|
| Molsidomin | 2 mg |
| Troxerutin | 10 mg |
| Maisstärke | 40 mg |
| Milchzucker | 107 mg |
| Magnesiumstearat | 1 mg |

Herstellung

20 g Molsidomin, 100 g Troxerutin, 400 g Maisstärke, 1070 g Milchzucker und 10 g Magnesiumstearat werden in einem Mischaggregat homogen gemischt, und dann wird die Mischung zu Tabletten von 160 mg Gewicht verpreßt.

Beispiel 8

Eine erfindungsgemäße, lichtstabilisierte Molsidomintablette hat beispielsweise folgende Zusammensetzung:

| | |
|---|---|
| Molsidomin | 8 mg |
| Troxerutin | 8 mg |
| Mikrokristalline Zellulose | 40 mg |
| Ricinusöl, hydriert | 30 mg |
| Milchzucker | 112 mg |
| Magnesiumstearat | 2 mg |

Herstellung

80 g Molsidomin, 80 g Troxerutin, 400 g mikrokristalline Zellulose, 300 g Ricinusöl, hydriert, und 1120 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpreßt.

Beispiel 9

Eine erfindungsgemäße, lichtstabilisierte Molsidomintablette hat beispielsweise folgende Zusammensetzung:

| | |
|---|---|
| Molsidomin | 16 mg |
| Troxerutin | 80 mg |
| Mikrokristalline Zellulose | 80 mg |
| Polyethylenglykol 6000 | 10 mg |
| Ricinusöl, hydriert | 50 mg |
| Milchzucker | 160 mg |
| Magnesiumstearat | 4 mg |

Herstellung

80 g Molsidomin, 400 g Troxerutin, 400 g mikrokristalline Zellulose, 50 g Polyethylenglykol 6000, 250 g Ricinusöl, hydriert, und 800 g Milchzucker werden bis zur homogenen Verteilung vorgemischt und dann in einem langsam laufenden Mischer mit Mantelheizung solange bei 70 °C weitergemischt, bis sich ein Granulat bildet. Dieses wird nach Abkühlen auf Zimmertemperatur durch ein 1,2 mm-Sieb passiert und nach Zusatz von 20 g Magnesiumstearat zu Tabletten von 400 mg Gewicht verpreßt.

Beispiel 10

Eine erfindungsgemäße, lichtstabilisierte Molsidomintablette hat beispielsweise folgende Zusammensetzung:

| | |
|---|---|
| Molsidomin | 8 mg |
| Troxerutin | 80 mg |
| Mikrokristalline Zellulose | 40 mg |
| Ricinusöl, hydriert | 30 mg |
| Milchzucker | 40 mg |
| Magnesiumstearat | 2 mg |

Herstellung

80 g Molsidomin, 800 g Troxerutin, 400 g mikrokristalline Zellulose, 300 g Ricinusöl, hydriert, 400 g Milchzucker und 20 g Magnesiumstearat werden in einem Mischaggregat homogen gemischt. Dann wird die Mischung zu Tabletten von 200 mg Gewicht verpreßt.

Beispiel 11

Molsidomin-Retardtabletten ohne und mit wechselnden Mengen Photostabilisator (Troxerutin) werden in folgender Zusammensetzung hergestellt:

| | | |
|---|---|---|
| a) | Molsidomin | 8 mg |
| | Mikrokristalline Zellulose | 40 mg |
| | Ricinusöl, hydriert | 30 mg |
| | Milchzucker | 120 mg |
| | Magnesiumstearat | 2 mg |

b), c), d)

| | |
|---|---|
| Molsidomin | 8 mg |
| Troxerutin | b) 8 mg, c) 0,8 mg; d) 0,08 mg |
| Mikrokristalline Zellulose | 40 mg |
| Ricinusöl, hydriert | 30 mg |
| Milchzucker | b) 112 mg; c) 119,2 mg; d) 120 mg |
| Magnesiumstearat | 2 mg |

Herstellung der Zubereitung a)

80 g Molsidomin, 400 g mikrokristalline Zellulose, 300 g Ricinusöl, hydriert, und 1200 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpreßt.

Herstellung der Zubereitungen b), c) und d)

80 g Molsidomin, b) 80 g, c) 8 g, d) 0,8 g Troxerutin, 400 g mikrokristalline Zellulose, 300 g Ricinusöl, hydriert, und b) 1120 g, c) 1192 g, d) 1200 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpreßt.

Die Tabletten der Zusammensetzung a), b), c) und d) werden der Belichtung in einem Suntest-Gerät der Firma Heraeus, Hanau, unterzogen und der Molsidominabbau gemessen. Die erhaltenen Molsidominmengen pro Tablette nach verschiedenen Belichtungszeiten sind der Fig. 3 zu entnehmen. Für einen 50%igen Molsidominabbau ergeben sich bei den verschiedenen Rezepturen die folgenden Belichtungszeiten:

a) 4,4 Std. b) 33,5 Std. c) 16,7 Std. d) 10,2 Std.

Man erkennt, daß in dieser festen Zubereitung eine Stabilisatorkonzentration von nur $^1/_{100}$ der Wirkstoffkonzentration noch eine erhebliche Photostabilisierung des Molsidomins bewirkt.

Beispiel 12

Molsidomin-Tropfen ohne und mit wechselnden Mengen Photostabilisator (Troxerutin) werden in folgender Zusammensetzung hergestellt.

| | |
|---|---|
| a) Molsidomin | 1,8 mg |
| Methylparaben | 0,7 mg |
| Propylparaben | 0,3 mg |
| entmineralisiertes Wasser ad | 1 ml |

b), c), d), e), f)

| | |
|---|---|
| Molsidomin | 1,8 mg |
| Troxerutin b) 18 mg, c) 1,8 mg, d) 0,18 mg, e) 0,036 mg, f) 0,0018 mg | |
| Methylparaben | 0,7 mg |
| Propylparaben | 0,3 mg |
| entmineralisiertes Wasser ad | 1 ml |

Herstellung der Zubereitung a)

In 3 Liter entmineralisiertem Wasser werden bei Siedetemperatur 3,5 g Methylparaben und 1,5 g Propylparaben gelöst. Nach dem Abkühlen auf Zimmertemperatur werden in dieser Lösung 9 g Molsidomin unter Rühren aufgelöst und das Volumen der Lösung durch Zusatz von entmineralisiertem Wasser auf 5 Liter eingestellt.

Herstellung der Zubereitungen b), c), d), e), f)

In 3 Liter entmineralisiertem Wasser werden bei Siedetemperatur 3,5 g Methylparaben und 1,5 g Propylparaben gelöst. Nach dem Abkühlen auf Zimmertemperatur werden in dieser Lösung 9 g Molsidomin und b) 90 g, c) 9 g, d) 0,9 g, e) 0,18 g, f) 0,009 g Troxerutin unter Rühren aufgelöst und das Volumen der Lösung durch Zusatz von entmineralisiertem Wasser auf 5 Liter eingestellt.

Die Tropfen der Zusammensetzung a) bis f) werden abgefüllt in Braunglasflaschen der Belichtung in einem Suntest-Gerät der Firma Heraeus, Hanau, unterzogen und der Molsidominabbau gemessen. Die erhaltenen Konzentrationswerte des Molsidomins nach verschiedenen Belichtungszeiten sind der Fig. 4 zu entnehmen. Folgende Regressionskoeffizienten des Molsidominabbaus ergeben sich bei den verschiedenen Rezepturen:

| | | |
|---|---|---|
| a) −5,19 | b) −0,07 | c) −0,42 |
| d) −1,07 | e) −1,69 | f) −2,16 |

Man erkennt, daß in dieser flüssigen Zubereitung eine Stabilisierungskonzentration von nur $^1/_{1000}$ der Wirkstoffkonzentration noch eine erhebliche Photostabilisierung des Molsidomins bewirkt.

Beispiel 13

Molsidomin-Tropfen ohne und mit wechselnden Mengen verschiedener Photostabilisatoren werden in folgender Zusammensetzung hergestellt:

Stabilisatorfreie Zubereitung:

| | |
|---|---|
| Molsidomin | 1,8 mg |
| Methylparaben | 0,7 mg |
| Propylparaben | 0,3 mg |
| entmineralisiertes Wasser ad | 1 ml |

Stabilisatorhaltige Zubereitungen:

| | |
|---|---|
| Molsidomin | 1,8 mg |
| Erfindungsgemäßer Photostabilisator | laut Tabelle Spalte A+B, |
| Methylparaben | 0,7 mg |
| Propylparaben | 0,3 mg |
| entmineralisiertes Wasser ad | 1 ml |

Herstellung

In 3 Liter entmineralisiertem Wasser werden bei Siedetemperatur 3,5 g Methylparaben und 1,5 g Propylparaben gelöst. Nach Abkühlen auf Zimmertemperatur werden in dieser Lösung 9 g Molsidomin und der erfindungsgemäße Photostabilisator laut Tabelle 1, Spalte A+C unter Rühren aufgelöst und das Volumen der Lösung durch Zusatz von entmineralisiertem Wasser auf 5 Liter eingestellt. Die so hergestellten Tropfen werden, abgefüllt in Braunglasflaschen, der Belichtung in einem Suntest-Gerät der Fa. Heraeus, Hanau, unterzogen, und der Molsidominabbau wird gemessen. Die hieraus errechneten Regressionskoeffizienten des Molsidominabbaus bei den verschiedenen Rezepturen sind der Spalte D der folgenden Tabelle 1 zu entnehmen.

Tabelle 1

| A Erfindungsgemäßer Photostabilisator | B Menge (mg) pro 1 ml d. Zubereitung | C Eingesetzte Menge (g) bei der Herstellung | D Regressions-koeffizient |
|---|---|---|---|
| Ohne Stabilisator | 0 | 0 | −5,19 |
| Mono-Rutin-schwefelsäureester (Na-Salz) | 1,8 | 9 | −0,27 |
| " | 0,18 | 0,9 | −2,29 |
| Mono-Hesperidin-phosphorsäureester (Na-Salz) | 1,8 | 9 | −1,61 |
| " | 0,18 | 0,9 | −2,43 |
| Hesperidin-methyl-chalcon | 18 | 90 | −1,48 |

Beispiel 14

In 100 ml n/100 Salzsäure wird 1 mg Molsidomin gelöst. Danach wird die Lösung halbiert. In der einen Hälfte der salzsauren Molsidominlösung werden 5 mg ®Venoruton einer standardisierten, handelsüblichen Mischung von O-(β-Hydroxyethyl)-rutosiden (HR), in der ca.

7–10% Tetra-5,7,3′,4′-HR
5–8% Tri-5,7,4′-HR
60–65% Tri-7,3′,4′-HR
8–12% Di-7,4′-HR und
5–8% Mono-4′HR

enthalten sind, aufgelöst.

Proben beider Lösungen werden, abgefüllt in farblose Probengläser, im Suntest-Gerät belichtet und die Abnahme der Molsidominkonzentration verfolgt.

Folgende Werte wurden gefunden:

| Lösung ohne ®Venoruton Zeit (min) | Konz. (% d. Anfangskonz.) | Lösung mit ®Venoruton Zeit (min) | Konz. (% d. Anfangskonz.) |
|---|---|---|---|
| 0,00 | 100,00 | 0,00 | 100,00 |
| 5,00 | 45,02 | 15,00 | 88,27 |
| 10,00 | 17,06 | 30,00 | 75,90 |
| 15,00 | 5,67 | 45,00 | 62,14 |
| 20,00 | 2,22 | 60,00 | 52,33 |
| 24,00 | 0,74 | 75,00 | 44,37 |
| | | 90,00 | 38,15 |
| | | 105,00 | 33,53 |
| | | 120,00 | 29,97 |

Aus diesen Werten errechnet sich eine Stabilitätserhöhung des Molsidomins um den Faktor 17,4.

Beispiel 15

Molsidomin-Tabletten mit einem erfindungsgemäßen Lichtschutz-Überzug werden wie folgt erhalten:

Zusammensetzung der Tablette:

| | |
|---|---|
| Molsidomin | 8 mg |
| Mikrokristalline Zellulose | 40 mg |
| Ricinusöl, hydriert | 30 mg |
| Milchzucker | 120 mg |
| Magnesiumstearat | 2 mg |

Herstellung

80 g Molsidomin, 400 g mikrokristalline Zellulose, 300 g Ricinusöl, hydriert, und 1200 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpreßt. Dann stellt man durch Auflösung von

80 g ®Methocel (Methylcellulose)
5 g Polyethylenglycol und
25 g Troxerutin oder
25 g Rutin in

690 ml demineralisiertem Wasser einen Lichtschutzlack her und sprüht damit Proben der obigen Tabletten so ein, daß nach dem Auftrocknen auf den Tabletten ein Film von ca. 4,4 mg pro Tablette entsteht, der 1 mg der Lichtschutzmittel, entweder Troxerutin oder Rutin, enthält.

Die lichtgeschützten Tabletten werden anschließend neben ungeschützten im Suntest-Gerät belichtet und die Abnahme des Molsidomingehalts bestimmt.

Folgende ausgezeichnete Ergebnisse wurden erhalten:

| Belichtungszeit (Std.) | Tablette ohne Lichtschutzfilm % d. Anf.konz. | Troxerutin-Film % d. Anf.konz. | Rutin-Film % d. Anf.konz. |
|---|---|---|---|
| 0 | 100 | 100 | 100 |
| 1 | 72,3 | 98,5 | 99,6 |
| 3 | 56,9 | 100,6 | 98,1 |
| 6 | 48,6 | 99,4 | 96,7 |
| 24 | 36,7 | 100,0 | 95,9 |

Beispiel 16

In 1 l eines 0,1 molaren Phosphatpuffers vom pH-Wert 7,0 werden 0,0826 mMol Molsidomin aufgelöst.

Zu je 100 ml dieser Lösung werden dann je 8,26 µMol der in der folgenden Tabelle 2 angegebenen, erfindungsgemäßen Photostabilisatoren aufgelöst.

Die Lösungen werden unter einem Braunfilter im «Suntest»-Gerät belichtet, die Molsidominkonzentration nach verschiedenen Belichtungszeiten bestimmt und der Regressionskoeffizient errechnet.

Folgende Werte wurden erhalten:

Tabelle 2
Molsidominkonzentrationen nach der Zeit

| Zeit Min. | ohne Photostabilisator | Hesperetin | Quercetin | Rutin | Quercitrin | Troxerutin |
|---|---|---|---|---|---|---|
| 0 | 100 | 100 | 100 | 100 | 100 | 100 |
| 15 | 64 | 88 | 95 | 90 | 89 | 89 |
| 30 | 35 | 76 | 87 | 81 | 79 | 79 |
| 45 | 14 | 65 | 74 | 71 | 67 | 69 |
| 60 | | 54 | | 61 | 56 | 60 |
| Regr. koeff (%) | −1,91 | −0,77 | −0,57 | −0,65 | −0,73 | −0,67 |

**Patentansprüche für die Vertragsstaaten BE, CH, DE, FR, GB, IT, LI, NL**

1. Photostabilisierte Mischung von Molsidomin, einem oder mehreren Photostabilisatoren und gegebenenfalls weiteren Mischungskomponenten, dadurch gekennzeichnet, daß die Photostabilisatoren Verbindungen der allgemeinen Formel I

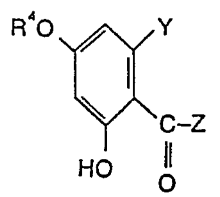

(I)

worin

Y eine Hydroxygruppe, Alkoxy mit 1 oder 2 C-Atomen oder β-Hydroxyethoxy und

Z eine Gruppe der Formel

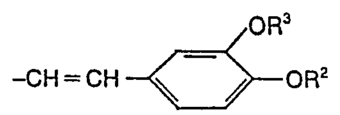

oder

Y und Z gemeinsam ein Strukturelement der Formel II oder III bedeuten

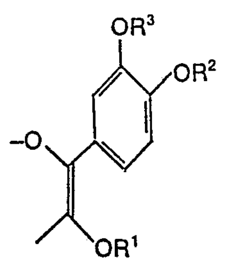

(II)

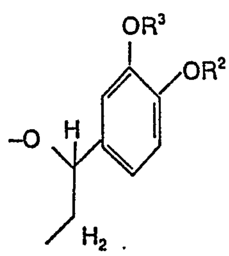

(III)

dessen freie Sauerstoffvalenz in der Y-Position und dessen freie Kohlenstoffvalenz in der Z-Position gebunden ist, und

$R^1$ Wasserstoff oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist, oder Ester dieser Verbindungen mit pharmakologisch unbedenklichen Säuren sowie gegebenenfalls dessen Salz mit pharmakologisch unbedenklichen Basen sind.

2. Photostabilisierte Mischung von Molsidomin gemäß Anspruch 1, dadurch gekennzeichnet, daß der Photostabilisator eine Verbindung Formel I ist, worin

Y, Z, $R^1$, $R^2$ und G die in Anspruch 1 genannten Bedeutungen haben,

$R^3$ Wasserstoff oder β-Hydroxyethyl und

$R^4$ Wasserstoff, β-Hydroxyethyl oder G bedeuten, oder ein Ester dieser Verbindungen mit pharmakologisch unbedenklichen Säuren sowie gegebenenfalls dessen Salz mit pharmakologisch unbedenklichen Basen ist.

3. Photostabilisierte Mischung von Molsidomin gemäß den Ansprüchen 1 und 2, dadurch gekennzeichnet, daß der Photostabilisator Hesperidin, Quercetin, Hesperetin, Quercitrin, Rutin oder Troxerutin oder ein Ester dieser Verbindungen mit pharmakologisch unbedenklichen Säuren sowie gegebenenfalls dessen Salz mit pharmakologisch unbedenklichen Basen ist.

4. Photostabilisierte Mischung von Molsidomin gemäß den Ansprüchen 1 bis 3, dadurch gekennzeichnet, daß der Photostabilisator Troxerutin ist.

5. Photostabilisierte Mischung von Molsidomin gemäß den Ansprüchen 1 bis 4, dadurch gekennzeichnet, daß darin Molsidomin und der Photostabilisator im Gewichtsverhältnis von 1:0,001 bis 1:50 vorliegen.

6. Photostabilisierte Mischung von Molsidomin gemäß den Ansprüchen 1 bis 4, dadurch gekennzeichnet, daß darin Molsidomin und der Photostabilisator im Gewichtsverhältnis von 1:0,3 bis 1:50 vorliegen.

7. Photostabilisierte pharmazeutische Zubereitung von Molsidomin, dadurch gekennzeichnet, daß sie als Photostabilisator eine Verbindung der allgemeinen Formel I

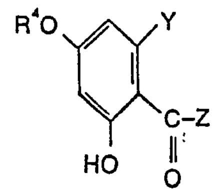

(I)

worin

Y eine Hydroxygruppe, Alkoxy mit 1 oder 2 C-Atomen oder β-Hydroxyethoxy und

Z eine Gruppe der Formel

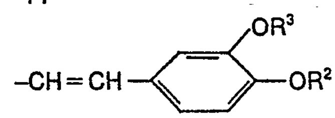

oder

Y und Z gemeinsam ein Strukturelement der Formel II oder III bedeuten

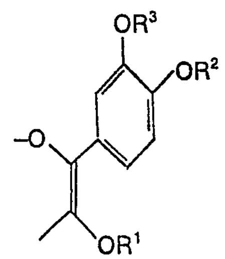

(II)

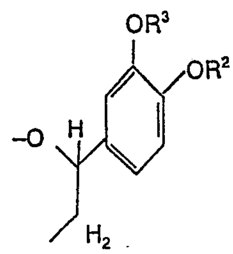

(III)

dessen freie Sauerstoffvalenz in der Y-Position und dessen freie Kohlenstoffvalenz in der Z-Position gebunden ist, und

$R^1$ Wasserstoff, oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist, oder einen Ester dieser Verbindungen mit pharmakologisch unbedenklichen Säuren sowie gegebenenfalls dessen Salz mit pharmakologisch unbedenklichen Basen enthält.

8. Photostabilisierte pharmazeutische Zubereitung gemäß Anspruch 7, dadurch gekennzeichnet, daß sie Wirkstoff und Photostabilisator im Gewichtsverhältnis von 1:0,001 bis 1:50 enthält.

9. Photostabilisierte pharmazeutische formstabile Zubereitung von Molsidomin gemäß Anspruch 7, dadurch gekennzeichnet, daß sie den Photostabilisator der im Anspruch 7 definierten Formel I in einem Überzug enthält.

10. Verwendung einer Verbindung der allgemeinen Formel I

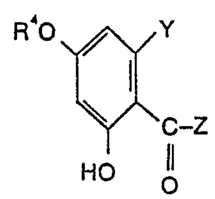

(I)

worin

Y eine Hydroxygruppe, Alkoxy mit 1 oder 2 C-Atomen oder β-Hydroxyethoxy und

Z eine Gruppe der Formel

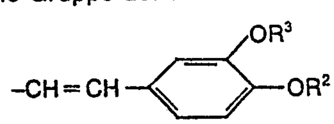

oder

Y und Z gemeinsam ein Strukturelement der Formel II oder III bedeuten

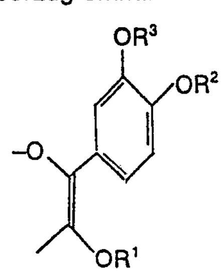

(II)

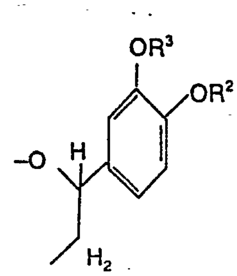

(III)

dessen freie Sauerstoffvalenz in der Y-Position und dessen freie Kohlenstoffvalenz in der Z-Position gebunden ist, und

$R^1$ Wasserstoff oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist oder eines Esters dieser Verbindung mit pharmakologisch unbedenklichen Säuren sowie dessen Salz mit pharmakologisch unbedenklichen Basen zur Photostabilisierung von Molsidomin.

11. Verwendung von Troxerutin zur Photostabilisierung von Molsidomin.

**Patentansprüche für den Vertragsstaat AT**

1. Verfahren zur Photostabilisierung von Molsidomin und von Mischungen von Molsidomin mit Mischungskomponenten, dadurch gekennzeichnet, daß man dem Molsidomin bzw. seinen Mischungen als Photostabilisator eine oder mehrere Verbindungen der allgemeinen Formel I

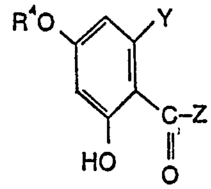

(I)

worin

Y eine Hydroxygruppe, Alkoxy mit 1 oder 2 C-Atomen oder β-Hydroxyethoxy und

Z eine Gruppe der Formel

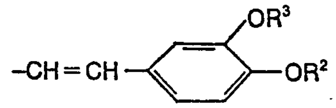

oder

Y und Z gemeinsam ein Strukturelement der Formel II oder III bedeuten

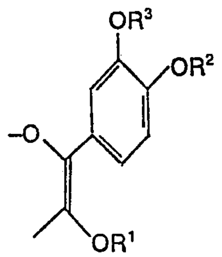

(II)

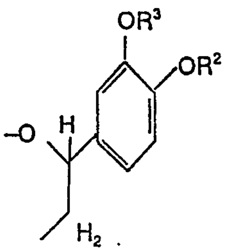

(III)

dessen freie Sauerstoffvalenz in der Y-Position und dessen freie Kohlenstoffvalenz in der Z-Position gebunden ist, und

$R^1$ Wasserstoff oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist, oder Ester dieser Verbindungen mit pharmakologisch unbedenklichen Säuren sowie gegebenenfalls dessen Salz mit pharmakologisch unbedenklichen Basen zusetzt.

2. Verfahren gemäß Anspruch 1, dadurch gekennzeichnet, daß man als Photostabilisator eine Verbindung der Formel I, worin

Y, Z, $R^1$, $R^2$ und G die im Anspruch 1 genannten Bedeutungen haben,

$R^3$ Wasserstoff oder β-Hydroxyethyl und

$R^4$ Wasserstoff, β-Hydroxyethyl oder G

bedeuten, oder einen Ester dieser Verbindungen mit pharmakologisch unbedenklichen Säuren sowie gegebenenfalls dessen Salz mit pharmakologisch unbedenklichen Basen zusetzt.

3. Verfahren gemäß Anspruch 1, dadurch gekennzeichnet, daß man als Photostabilisator Hesperidin, Quercetin, Hesperetin, Quercitrin, Rutin oder Troxerutin oder einen Ester dieser Verbindungen mit pharmakologisch unbedenklichen Säuren sowie gegebenenfalls dessen Salz mit pharmakologisch unbedenklichen Basen zusetzt.

4. Verfahren gemäß Anspruch 1, dadurch gekennzeichnet, daß man als Photostabilisator Troxerutin zusetzt.

5. Verfahren gemäß den Ansprüchen 1 bis 4, dadurch gekennzeichnet, daß man, bezogen auf Molsidomin, den Photostabilisator im Gewichtsverhältnis von 1:0,001 bis 1:50 zusetzt.

6. Verfahren gemäß den Ansprüchen 1 bis 5, dadurch gekennzeichnet, daß man, bezogen auf Molsidomin, den Photostabilisator im Gewichtsverhältnis von 1:0,3 bis 1:50 zusetzt.

7. Verfahren gemäß Anspruch 1 zur Photostabilisierung von pharmazeutischen Zubereitungen von Molsidomin, die neben dem Molsidomin noch Hilfsstoffe, wie sie in der Galenik üblich sind, enthalten, dadurch gekennzeichnet, daß man das Molsidomin mit den Hilfsstoffen und einem Photostabilisator der im Anspruch 1 definierten Formel I mischt und in an sich bekannter Weise in die galenische Anwendungsform überführt.

8. Verfahren zur Photostabilisierung von formstabilen galenischen Zubereitungen von Molsidomin, dadurch gekennzeichnet, daß man sie mit einem Überzug versieht, der einen Photostabilisator der im Anspruch 1 definierten Formel I enthält.

9. Verwendung einer Verbindung der allgemeinen Formel I

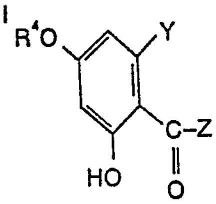

(I)

worin

Y eine Hydroxygruppe, Alkoxy mit 1 oder 2 C-Atomen oder β-Hydroxyethoxy und

Z eine Gruppe der Formel

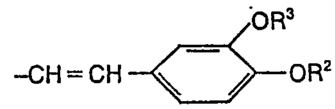

oder

Y und Z gemeinsam ein Strukturelement der Formel II oder III bedeuten

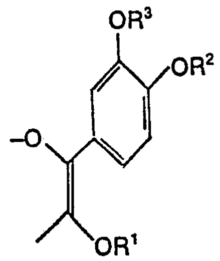

(II)

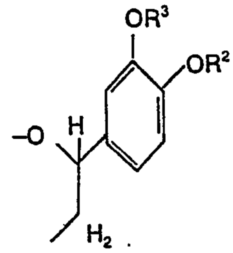

(III)

dessen freie Sauerstoffvalenz in der Y-Position und dessen freie Kohlenstoffvalenz in der Z-Position gebunden ist, und

$R^1$ Wasserstoff oder einen Rest G,

$R^2$ Wasserstoff, Alkyl mit 1 oder 2 C-Atomen oder β-Hydroxyethyl,

$R^3$ Wasserstoff, Alkyl mit 1 oder 2 C-Atomen oder β-Hydroxyethyl,

$R^4$ Wasserstoff, Alkyl mit 1 oder 2 C-Atomen, β-Hydroxyethyl oder einen Rest G bedeuten und

G der Rest eines Mono- oder Disaccharids ist oder eines Esters dieser Verbindung mit pharmakologisch unbedenklichen Säuren sowie dessen Salz mit pharmakologisch unbedenklichen Basen zur Photostabilisierung von Molsidomin.

10. Verwendung von Troxerutin zur Photostabilisierung von Molsidomin.

**Claims for the Contracting States: BE, CH, DE, FR, GB, IT, LI, NL**

1. Photostabilised mixture of molsidomine, one or several photostabilisers, and, if appropriate, further mixing components, characterised in that the photostabilisers are compounds of the general formula I

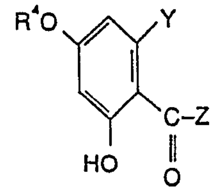

(I)

wherein Y denotes a hydroxyl group, alkoxy having 1 or 2 C atoms or β-hydroxyethoxy and Z denotes a group of the formula

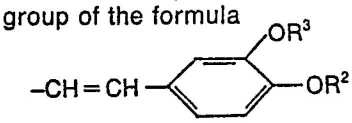

or Y and Z together denote a structural element of the formula II or III

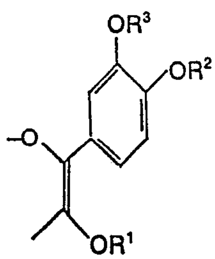

(II)

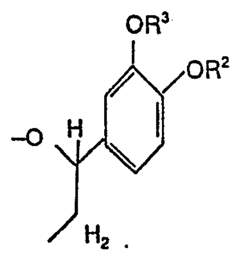

(III)

in which the free oxygen valency is attached in the Y-position and the free carbon valency is attached in the Z-position and $R^1$ denotes hydrogen or a radical G, $R^2$ denotes hydrogen, alkyl having 1 or 2 C atoms or β-hydroxyethyl, $R^3$ denotes hydrogen, alkyl having 1 or 2 C atoms or β-hydroxyethyl, $R^4$ denotes hydrogen, alkyl having 1 or 2 C atoms, β-hydroxyethyl or a radical G and G is the radical of a monosaccharide or disaccharide, or esters of these compounds with pharmacologically acceptable acids and also, if appropriate, a salt thereof with pharmacologically acceptable bases.

2. Photostabilised mixture of molsidomine according to Claim 1, characterised in that the photostabiliser is a compound of the formula I wherein Y, Z, $R^1$, $R^2$ and G have the meanings mentioned in Claim 1, $R^3$ denotes hydrogen or β-hydroxyethyl and $R^4$ denotes hydrogen, β-hydroxyethyl or G, or is an ester of these compounds with pharmacologically acceptable acids and also, if appropriate a salt thereof with pharmacologically acceptable bases.

3. Photostabilised mixture of molsidomine according to Claims 1 and 2, characterised in that the

photostabiliser is hesperidin, quercetin, hesperitin, quercitrin, rutin or troxerutin or an ester of these compounds with pharmacologically acceptable acids and also, if appropriate, a salt thereof with pharmacologically acceptable bases.

4. Photostabilised mixture of molsidomine according to Claims 1 to 3, characterised in that the photostabiliser is troxerutin.

5. Photostabilised mixture of molsidomine according to Claims 1 to 4, characterised in that molsidomine and the photostabiliser are present therein in a ratio by weight of 1:0.001 to 1:50.

6. Photostabilised mixture of molsidomine according to Claims 1 to 4, characterised in that molsidomine and the photostabiliser are present therein in a ratio by weight of 1:0.3 to 1:50.

7. Photostabilised pharmaceutical formulation of molsidomine, characterised in that it contains, as the photostabiliser, a compound of the general formula I

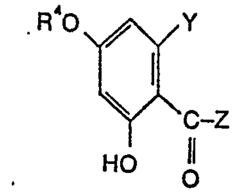

(I)

wherein Y denotes a hydroxyl group, alkoxy having 1 or 2 C atoms or β-hydroxyethoxy and Z denotes a group of the formula

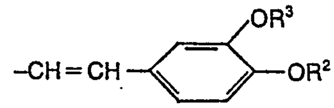

or Y and Z together denote a structural element of the formula II or III

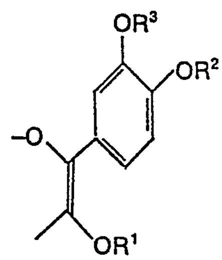

(II)

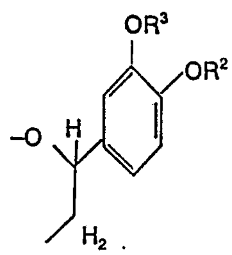

(III)

in which the free oxygen valency is attached in the Y-position and the free carbon valency is attached in the Z-position and $R^1$ denotes hydrogen or a radical G, $R^2$ denotes hydrogen, alkyl having 1 or 2 C atoms or β-hydroxyethyl, $R^3$ denotes hydrogen, alkyl having 1 or 2 C atoms or β-hydroxyethyl, $R^4$ denotes hydrogen, alkyl having 1 or 2 C atoms, β-hydroxyethyl or a radical G and G is the radical of a monosaccharide or disaccharide, or an ester of these compounds with pharmacologically acceptable acids and also, if appropriate, a salt thereof with pharmacologically acceptable bases.

8. Photostabilised pharmaceutical formulation of molsidomine according to Claim 7, characterised in that it contains the acive compound and photostabiliser in a ratio by weight of 1:0.001 to 1:50.

9. Photostabilised pharmaceutical dimensionally stable formulation according to Claim 7, characterised in that it contains the photostabiliser of the formula I defined in Claim 7 in a coating.

10. The use of a compound of the genera formula I

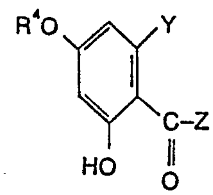

(I)

wherein Y denotes a hydroxyl group, alkoxy having 1 or 2 C atoms or β-hydroxyethoxy and Z denotes a group of the formula

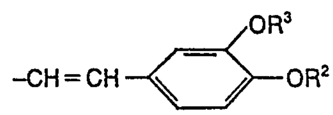

or Y and Z together denote a structural element of the formula II or III

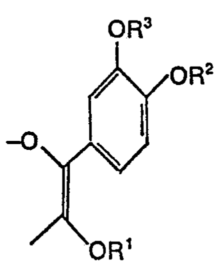

(II)

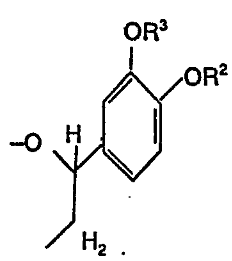

(III)

in which the free oxygen valency is attached in the Y-position and the free carbon valency is attached in the Z-position and $R^1$ denotes hydrogen or a radical G, $R^2$ denotes hydrogen, alkyl having 1 or 2 C atoms or β-hydroxyethyl, $R^3$ denotes hydrogen, alkyl having 1 or 2 C atoms or β-hydroxyethyl, $R^4$ denotes hydrogen, alkyl having 1 or 2 C atoms, β-hydroxyethyl or a radical G and G is the radical of a monosaccharide or disaccharide, or of an ester of this compound with pharmacologically

acceptable acids and also a salt thereof with pharmacologically acceptable bases for photostabilising molsidomine.

11. The use of troxerutin for photostabilising molsidomine.

**Claims for the Contracting State: AT**

1. Process for photostabilising molsidomine and mixtures of molsidomine with mixing components, characterised in that there are added, respectively to the molsidomine and its mixtures, as photostabilisers, one or several compounds of the general formula I

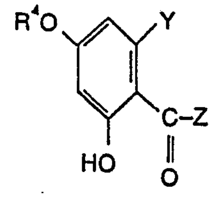

(I)

wherein Y denotes a hydroxyl group, alkoxy having 1 or 2 C atoms or β-hydroxyethoxy and Z denotes a group of the formula

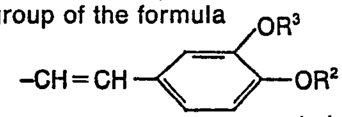

or Y and Z together denote a structural element of the formula II or III

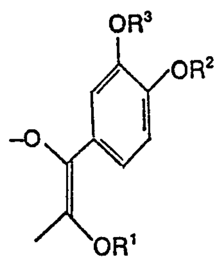

(II)

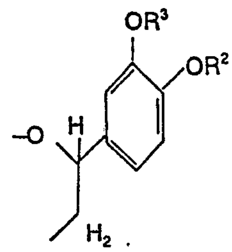

(III)

in which the free oxygen valency is attached in the Y-position and the free carbon valency is attached in the Z-position and $R^1$ denotes hydrogen or a radical G, $R^2$ denotes hydrogen, alkyl having 1 or 2 C atoms or β-hydroxyethyl, $R^3$ denotes hydrogen, alkyl having 1 or 2 C atoms or β-hydroxyethyl, $R^4$ denotes hydrogen, alkyl having 1 or 2 C atoms, β-hydroxyethyl or a radical G and G is the radical of a monosaccharide or disaccharide, or esters of these compounds with pharmacologically acceptable acids and also, if appropriate, a salt thereof with pharmacologically acceptable bases.

2. Process according to Claim 1, characterised in that the added photostabiliser is a compound of the formula I wherein Y, Z, $R^1$, $R^2$ and G have the meanings mentioned in Claim 1, $R^3$ denotes hydrogen or β-hydroxyethyl and $R^4$ denotes hydrogen, β-hydroxyethyl or G, or is an ester of these compounds with pharmacologically acceptable acids and also, if appropriate, a salt thereof with pharmacologically acceptable bases.

3. Process according to Claim 1, characterised in that the photostabiliser is hesperidin, quercetin, hesperitin, quercitrin, rutin or troxerutin or an ester of these compounds with pharmacologically acceptable acids and also, if appropriate, a salt thereof with pharmacologically acceptable bases.

4. Process according to Claim 1, characterised in that the added photostabiliser is troxerutin.

5. Process according to Claims 1 to 4, characterised in that the photostabiliser is added in a ratio by weight of 1:0.001 to 1:50, relative to molsidomine.

6. Process according to Claims 1 to 5, characterised in that the photostabiliser is added in a ratio by weight of 1:0.3 to 1:50, relative to molsidomine.

7. Process according to Claim 1 for photostabilising pharmaceutical formulations of molsidomine which contain, in addition to the molsidomine, galenically customary adjuvants, characterised in that the molsidomine is mixed with the adjuvants and a photostabiliser defined in formula I of Claim 1 and the mixture is converted in a manner known per se into the galenical form of application.

8. Process for photostabilising dimensionally stable galenical formulations of molsidomine, characterised in that they are provided with a coating which contains a photostabiliser of the formula I defined in Claim 1.

9. The use of a compound of the general formula I

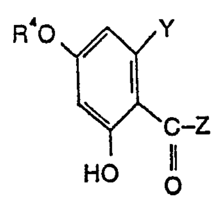

(I)

wherein Y denotes a hydroxyl group, alkoxy having 1 or 2 C atoms or β-hydroxyethoxy and Z denotes a group of the formula

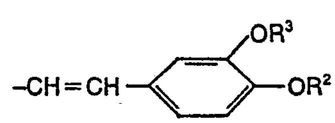

or Y and Z together denote a structural element of the formula II or III

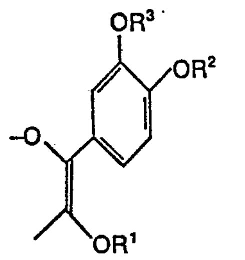

(II)

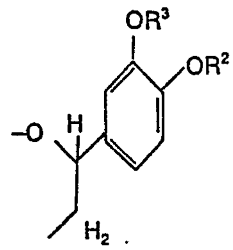

(III)

in which the free oxygen valency is attached in the Y-position and the free carbon valency is attached in the Z-position and $R^1$ denotes hydrogen or a radical G, $R^2$ denotes hydrogen, alkyl having 1 or 2 C atoms or β-hydroxyethyl, $R^3$ denotes hydrogen, alkyl having 1 or 2 C atoms or β-hydroxyethyl, $R^4$ denotes hydrogen, alkyl having 1 or 2 C atoms, β-hydroxyethyl or a radical G and G is the radical of a monosaccharide or disaccharide, or of an ester of this compound with pharmacologically acceptable acids and also a salt thereof with pharmacologically acceptable bases for photostabilising molsidomine.

10. The use of troxerutin for photostabilising molsidomine.

**Revendications pour les Etats contractants: BE, CH, DE, FR, GB, IT, LI, NL**

1. Mélange photostabilisé contenant de la molsidomine, un ou plusieurs photostabilisants et, éventuellement, d'autres constituants, mélange caractérisé en ce que les photostabilisants sont des composés répondant à la formule générale I:

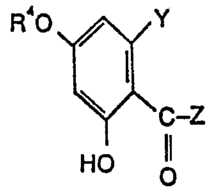

(I),

dans laquelle

Y représente un radical hydroxy, un alcoxy contenant un ou deux atomes de carbone ou un hydroxy-2 éthoxy,

Z représente un radical de formule:

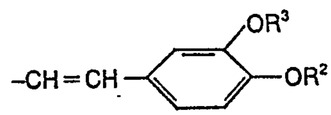

ou

Y et Z forment ensemble un élément structural répondant à l'une des formules II et III

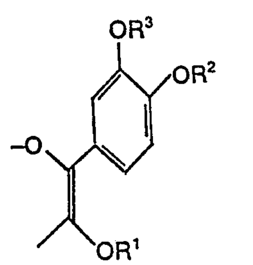

(II)

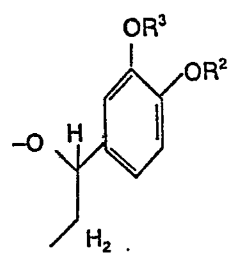

(III)

dont la valence libre partant de l'oxygène est liée à la position de Y et dont la valence libre partant du carbone est liée à la position de Z,

$R^1$ représente l'hydrogène ou un radical G,

$R^2$ représente l'hydrogène, un alkyle à un ou deux atomes de carbone ou un hydroxy-2 éthyle,

$R^3$ représente l'hydrogène, un alkyle à un ou deux atomes de carbone ou un hydroxy-2 éthyle,

$R^4$ représente l'hydrogène, un alkyle à un ou deux atomes de carbone, un hydroxy-2 éthyle ou un radical G et

G représente le radical d'un monosaccharide ou d'un disaccharide,

ou des esters formés par ces composés avec des acides acceptables du point de vue pharmacologique, ainsi qu'éventuellement leurs sels avec des bases acceptables du point de vue pharmacologique.

2. Mélange photostabilisé de molsidomine selon la revendication 1, caractérisé en ce que le photostabilisant est un composé de formule I dans lequel:

Y, Z, $R^1$, $R^2$ et G ont les significations qui leur ont été données à la revendication 1,

$R^3$ représente l'hydrogène ou un radical hydroxy-2 éthyle et

$R^4$ représente l'hydrogène, un radical hydroxy-2 éthyle ou un radical G,

ou un ester dérivant d'un tel composé et d'un acide acceptable du point de vue pharmacologique, et éventuellement un sel d'un tel composé avec une base acceptable du point de vue pharmacologique.

3. Mélange photostabilisé de molsidomine selon l'une des revendications 1 et 2, mélange caractérisé en ce que le photostabilisant est l'hespéridine, la quercétine, l'hespérétine, la quercitrine, la rutine ou la troxérutine ou un ester formé par un tel composé avec un acide acceptable du point de vue pharmacologique, et éventuellement un sel d'un tel composé avec une base acceptable du point de vue pharmacologique.

4. Mélange photostabilisé de molsidomine selon l'une quelconque des revendications 1 à 3,

caractérisé en ce que le photostabilisant est la troxérutine.

5. Mélange photostabilisé de molsidomine selon l'une quelconque des revendications 1 à 4, caractérisé en ce que la molsidomine et le photostabilisant sont présents en un rapport pondéral compris entre 1:0,001 et 1:50.

6. Mélange photostabilisé de molsidomine selon l'une quelconque des revendications 1 à 4. caractérisé en ce que la molsidomine et le photostabilisant sont présents en un rapport pondéral compris entre 1:0,3 et 1:50.

7. Composition pharmaceutique de molsidomine photostabilisée, caractérisée en ce qu'elle contient, comme photostabilisant, un composé répondant à la formule générale I:

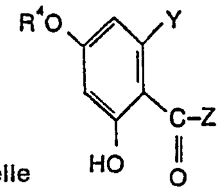

(I)

dans laquelle

Y représente un radical hydroxy, un alcoxy contenant un ou deux atomes de carbone ou un hydroxy-2 éthoxy,

Z représente un radical de formule:

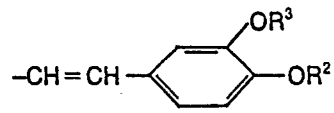

ou

Y et Z forment ensemble un élément structural répondant à l'une des formules II et III

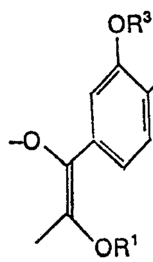

(II)

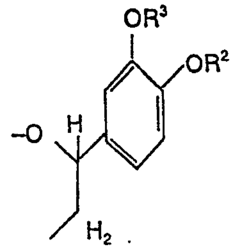

(III)

dont la valence libre partant de l'oxygène est liée à la position de Y et dont la valence libre partant du carbone est liée à la position de Z,

$R^1$ représente l'hydrogène ou un radical G,

$R^2$ représente l'hydrogène, un alkyle à un ou deux atomes de carbone ou un hydroxy-2 éthyle,

$R^3$ représente l'hydrogène, un alkye à un ou deux atomes de carbone ou un hydroxy-2 éthyle,

$R^4$ représente l'hydrogène, un alkyle à un ou deux atomes de carbone, un hydroxy-2 éthyle ou un radical G et

G représente le radical d'un monosaccharide ou d'un disaccharide,

ou un ester formé par ce composé avec un acide acceptable du point de vue pharmacologique, ainsi qu'éventuellement l'un des sels avec des bases acceptables du point de vue pharmacologique.

8. Composition pharmaceutique photostabilisée selon la revendication 7, caractérisée en ce qu'elle contient la substance active et le photostabilisant dans un rapport pondéral compris entre 1:0,001 et 1:50.

9. Composition pharmaceutique de molsidomine, photostabilisée et de forme stable, selon la revendication 7, caractérisée en ce qu'elle contient le photostabilisant de formule I qui a été défini à la revendication 7 dans un revêtement.

10. Application, pour la photostabilisation de la molsidomine, d'un composé répondant à la formule générale I:

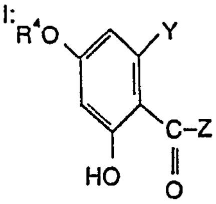

(I)

dans laquelle

Y représente un radical hydroxy, un alcoxy contenant un ou deux atomes de carbone ou un hydroxy-2 éthoxy,

Z représente un radical de formule:

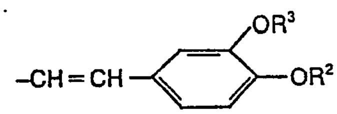

ou

Y et Z forment ensemble un élément structural répondant à l'une des formules II et III:

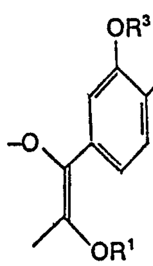

(II)

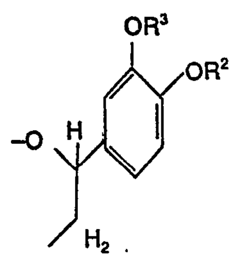

(III)

dont la valence libre partant de l'oxygène est liée à la position de Y et dont la valence libre partant du carbone est liée à la position de Z,

$R^1$ représente l'hydrogène ou un radical G,

$R^2$ représente l'hydrogène, un alkyle à un ou deux atomes de carbone ou un hydroxy-2 éthyle,

$R^3$ représente l'hydrogène, un alkyle à un ou deux atomes de carbone ou un hydroxy-2 éthyle,

$R^4$ représente l'hydrogène, un alkyle à un ou deux atomes de carbone, un hydroxy-2 éthyle ou un radical G et

G représente le radical d'un monosaccharide ou d'un disaccharide,

ou d'un ester formé par ce composé avec un acide acceptable du point de vue pharmacologique, ainsi qu'éventuellement d'un de ses sels avec des bases acceptables du point de vue pharmacologique.

11. Application de la troxérutine pour la photostabilisation de la molsidomine.

**Revendications pour l'Etat contractant: AT**

1. Procédé pour photostabiliser la molsidomine et des mélanges de molsidomine avec d'autres constituants, procédé caractérisé en ce qu'on ajoute à la molsidomine ou à ses mélanges, comme photostabilisant, un ou plusieurs composés répondant à la formule générale I:

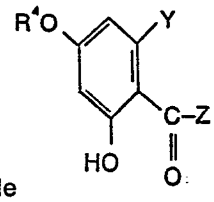

(I)

dans laquelle

Y représente un radical hydroxy, un alcoxy contenant un ou deux atomes de carbone ou un hydroxy-2 éthoxy,

Z représente un radical de formule:

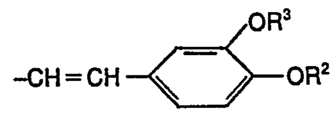

ou

Y et Z forment ensemble un élément structural répondant à l'une des formules II et III

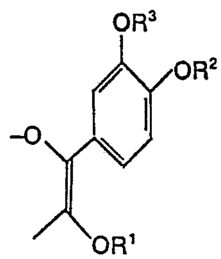

(II)

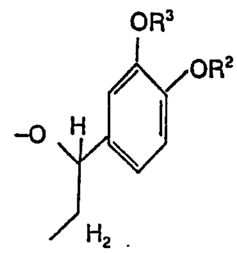

(III)

dont la valence libre partant de l'oxygène est liée à la position de Y et dont la valence libre partant du carbone est liée à la position de Z,

$R^1$ représente l'hydrogène ou un radical G,

$R^2$ représente l'hydrogène, un alkyle à un ou deux atomes de carbone ou un hydroxy-2 éthyle,

$R^3$ représente l'hydrogène, un alkyle à un ou deux atomes de carbone ou un hydroxy-2 éthyle,

$R^4$ représente l'hydrogène, un alkyle à un ou deux atomes de carbone, un hydroxy-2 éthyle ou un radical G et

G représente le radical d'un monosaccharide ou d'un disaccharide,

ou des esters formés par ces composés avec des acides acceptables du point de vue pharmacologique, ainsi qu'éventuellement leurs sels avec des bases acceptables du point de vue pharmacologique.

2. Procédé selon la revendication 1, caractérisé en ce qu'on ajoute, comme photostabilisant, un composé de formule I dans lequel:

Y, Z, $R^1$, $R^2$ et G ont les significations qui leur ont été données à la revendication 1,

$R^3$ représente l'hydrogène ou un radical hydroxy-2 éthyle et

$R^4$ représente l'hydrogène, un radical hydroxy-2 éthyle ou un radical G,

ou un ester dérivant d'un tel composé et d'un acide acceptable du point de vue pharmacologique, et éventuellement un sel d'un tel composé avec une base acceptable du point de vue pharmacologique.

3. Procédé selon la revendication 1, caractérisé en ce qu'on ajoute, comme photostabilisant, de l'hespéridine, del la quercétine, de l'hespérétine, de la quercitrine, de la rutine ou de la troxérutine, ou un ester dérivant d'un tel composé et d'un acide acceptable du point de vue pharmacologique, ou un sel formé par un tel composé avec une base acceptable d'un point de vue pharmacologique.

4. Procédé selon la revendication 1, caractérisé en ce qu'on ajoute de la troxérutine comme photostabilisant.

5. Procédé selon l'une quelconque des revendications 1 à 4, caractérisé en ce qu'on ajoute le photostabilisant dans un rapport pondéral, relativement à la molsidomine, compris entre 1:0,001 et 1:50.

6. Procédé selon l'une quelconque des revendications 1 à 5, caractérisé en ce qu'on ajoute le photostabilisant dans un rapport pondéral, relativement à la molsidomine, compris entre 1:0,3 et 1:50.

7. Procédé selon la revendication 1 pour la photostabilisation de compositions pharmaceutiques à base de molsidomine qui, en plus de la molsidomine, contiennent des adjuvants, tels que ceux qui sont couramment utilisés en pharmacie, procédé caractérisé en ce qu'on mélange la molsidomine avec les adjuvants et un photostabilisant de formule I tel que défini à la revendication 1, et on met le mélange, de manière connue, sous une forme d'application pharmaceutique.

8. Procédè pour photostabiliser des compositions pharmaceutiques de molsidomine de forme stable, procédé caractérisé en ce qu'on les munit

d'un revêtement qui contient un photostabilisant de formule I selon la revendication 1.

9. Application, pour la photostabilisation de la molsidomine, d'un composé répondant à la formule générale I:

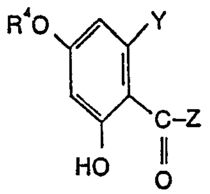

(I)

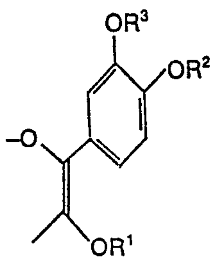

(II)

dont la valence libre partant de l'oxygène est liée à la position de Y et dont la valence libre partant du carbone est liée à la position de Z,

$R^1$ représente l'hydrogène ou un radical G,

$R^2$ représente l'hydrogène, un alkyle à un ou deux atomes de carbone ou un hydroxy-2 éthyle,

$R^3$ représente l'hydrogène, un alkyle à un ou deux atomes de carbone ou un hydroxy-2 éthyle,

$R^4$ représente l'hydrogène, un alkyle à un ou deux atomes de carbone, un hydroxy-2 éthyle ou un radical G et

dans laquelle

Y représente un radical hydroxy, un alcoxy contenant un ou deux atomes de carbone ou un hydroxy-2 éthoxy,

Z représente un radical de formule:

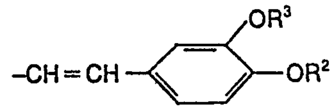

ou

Y et Z forment ensemble un élément structural répondant à l'une des formules II et III

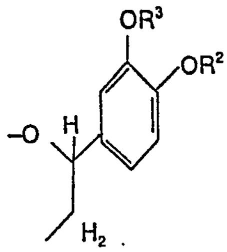

(III)

G représente le radical d'un monosaccharide ou d'un disaccharide,

ou d'un ester formé par ce composé avec un acide acceptable du point de vue pharmacologique, ainsi qu'éventuellement d'un de ses sels avec des bases acceptables du point de vue pharmacologique.

10. Application de la troxérutine pour la photostabilisation de la molsidomine.

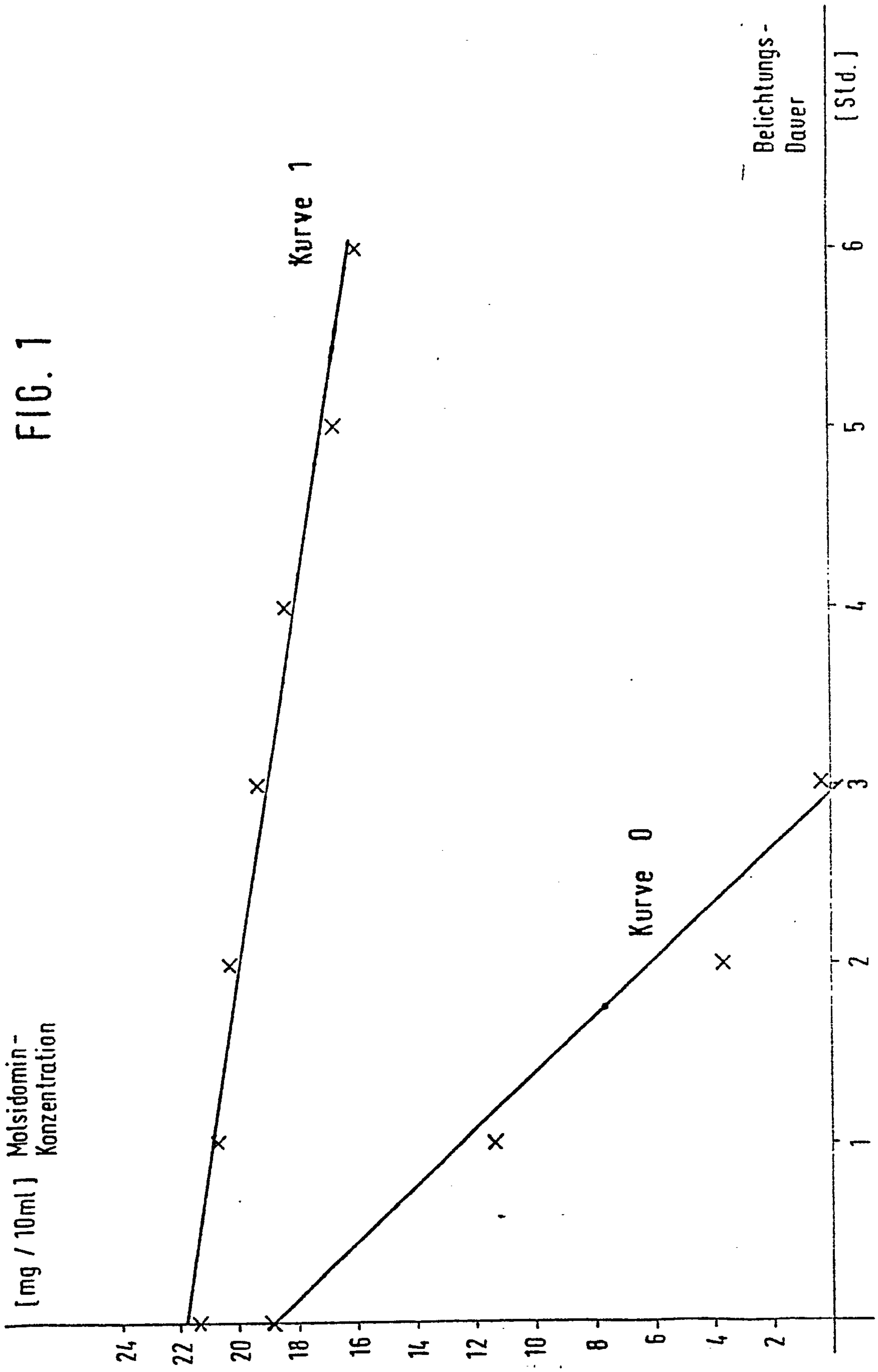
[mg / 10ml] Molsidomin-
Konzentration
Kurve 1
Kurve 0
Belichtungs-
Dauer
[Std.]
24
22
20
18
16
14
12
10
8
6
4
2
1
2
3
4
5
6

FIG. 1

FIG. 2

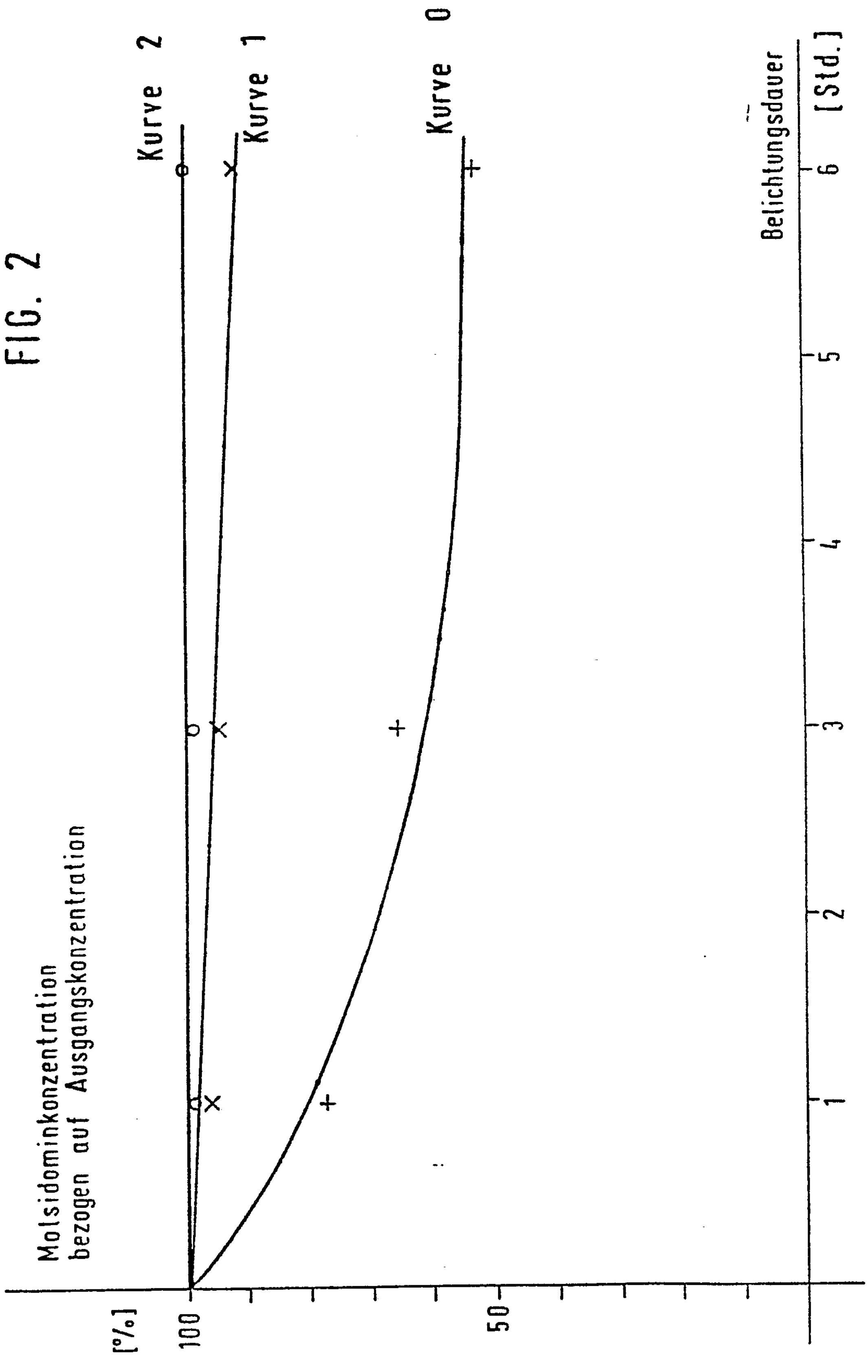
Molsidominkonzentration
bezogen auf Ausgangskonzentration
[%]
100
50
Kurve 2
Kurve 1
Kurve 0
1
2
3
4
5
6
Belichtungsdauer [Std.]

FIG. 3

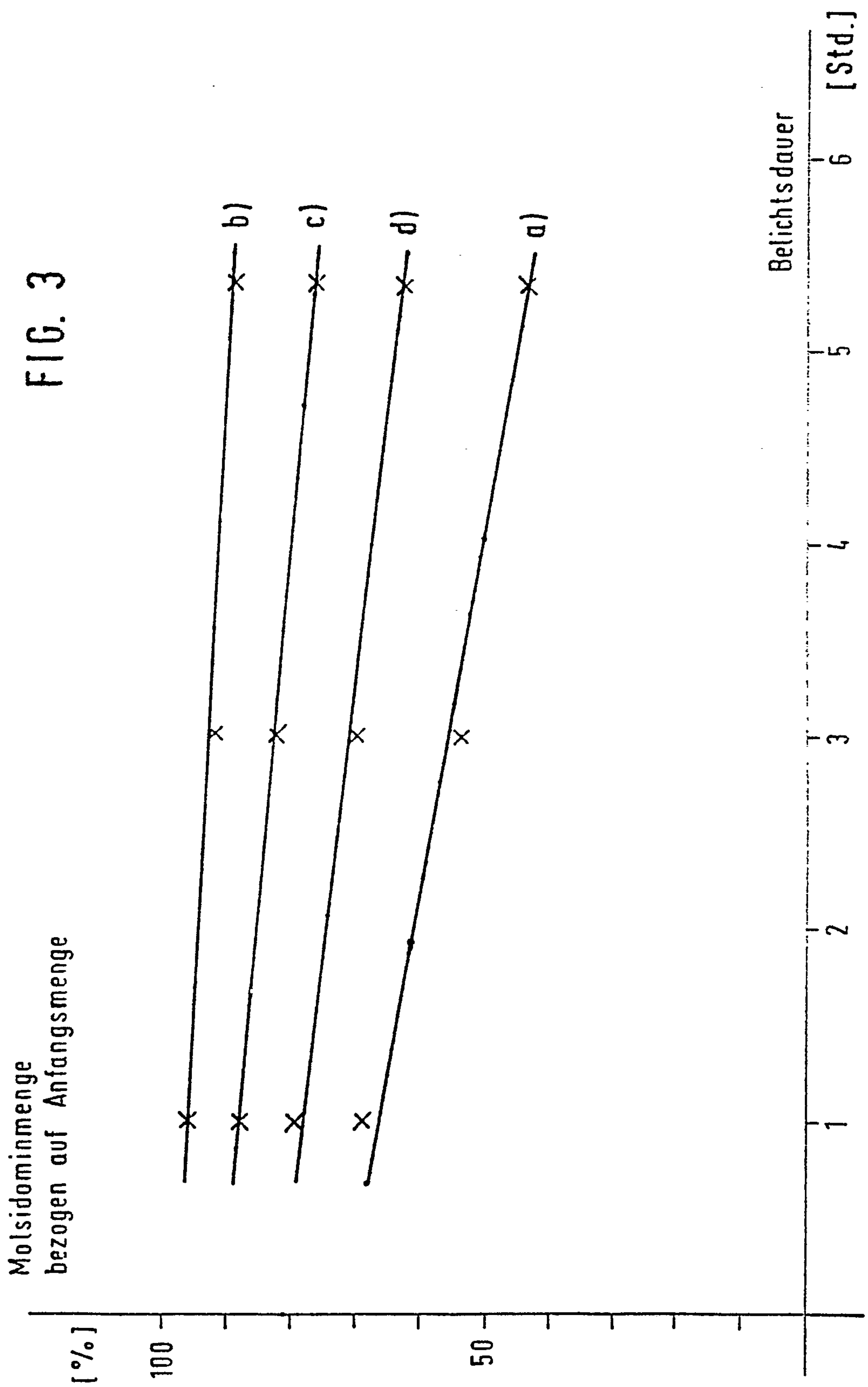
Molsidominmenge
bezogen auf Anfangsmenge
[%]
100
50
Belichtsdauer
[Std.]
1
2
3
4
5
6
a)
b)
c)
d)

FIG. 4

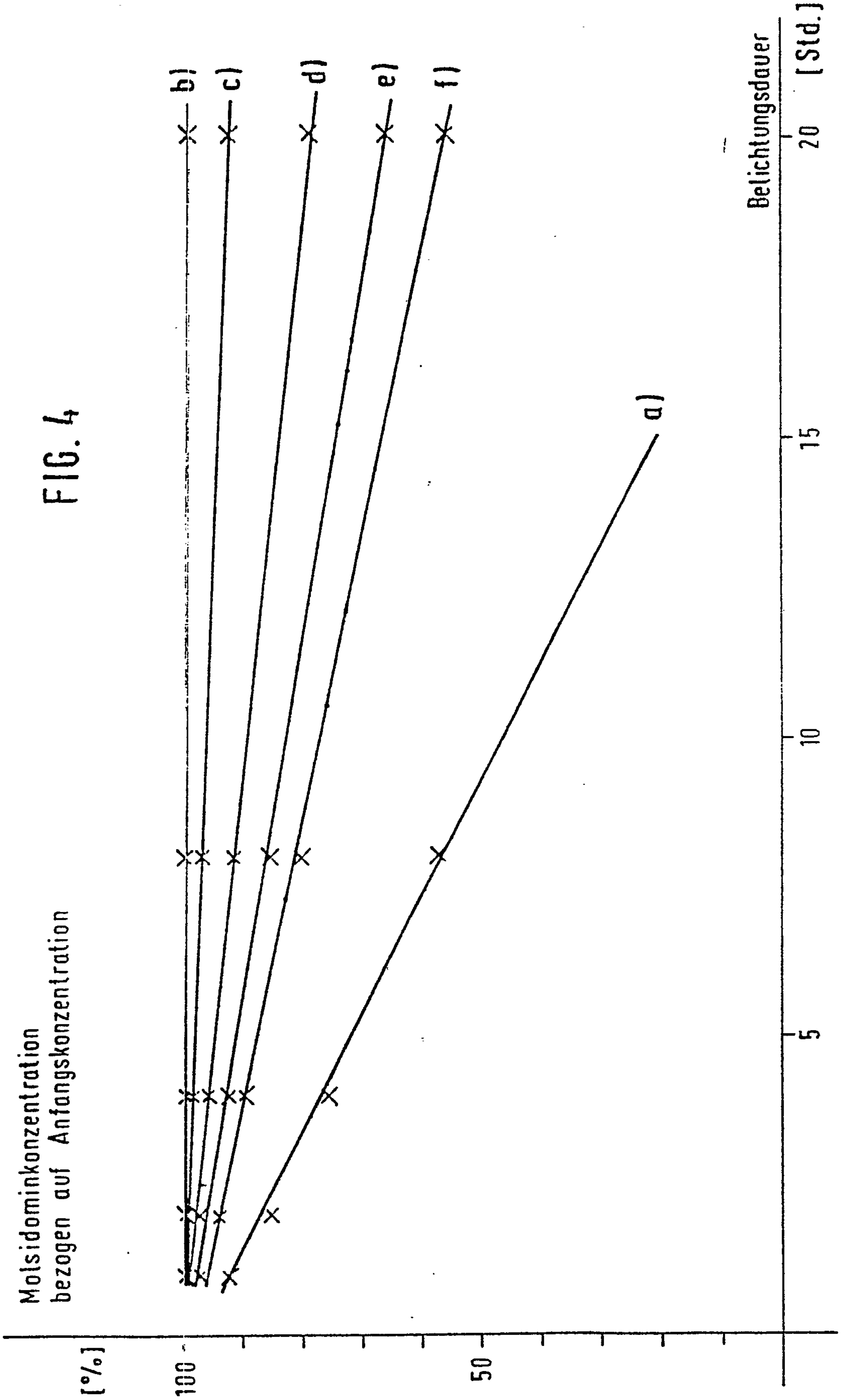
Molsidominkonzentration
bezogen auf Anfangskonzentration
[%]
100
50
Belichtungsdauer
[Std.]
5
10
15
20
a)
b)
c)
d)
e)
f)